Europäisches Patentamt

European Patent Office

Office européen des brevets

(19)

(11) Veröffentlichungsnummer : **0 124 506
B1**

# EUROPÄISCHE PATENTSCHRIFT

(12)

(45) Veröffentlichungstag der Patentschrift :
17.08.88

(21) Anmeldenummer : 84890074.2

(22) Anmeldetag : 26.04.84

(51) Int. Cl.⁴ : **A 61 L   2/04// A61K35/16**

(54) Verfahren zur Inaktivierung von vermehrungsfähigen Krankheitserregern.

(30) Priorität : 02.05.83 AT 1593/83
02.05.83 AT 1592/83
02.05.83 AT 1591/83
02.05.83 AT 1590/83

(43) Veröffentlichungstag der Anmeldung :
07.11.84 Patentblatt 84/45

(45) Bekanntmachung des Hinweises auf die Patenterteilung : 17.08.88 Patentblatt 88/33

(84) Benannte Vertragsstaaten :
AT BE CH DE FR GB IT LI NL SE

(56) Entgegenhaltungen :
EP-A- 0 011 739
WO-A-82 /038 71
US-A- 4 137 307

(73) Patentinhaber : IMMUNO Aktiengesellschaft für che-
misch-medizinische Produkte
Industriestrasse 72
A-1220 Wien (AT)

(72) Erfinder : Philapitsch, Anton
Hans Kudlichgasse 5
A-2490 Ebenfurt (AT)
Erfinder : Wöber, Günter, Prof. Dr.
Carolusstrasse 23
A-2522 Oberwaltersdorf (AT)
Erfinder : Eibl, Johann, Dr.
Gustav Tschermakgasse 2
A-1180 Wien (AT)
Erfinder : Schwarz, Otto, Dr.
Celtesgasse 5
A-1190 Wien (AT)

(74) Vertreter : Wolfram, Gustav, Dipl.-Ing.
Schwindgasse 7 P.O. Box 205
A-1041 Wien (AT)

**Beschreibung**

Die Erfindung betrifft ein Verfahren zur Inaktivierung von vermehrungsfähigen Krankheitserregern in Präparationen, die plasmatische Enzyme und/oder Proenzyme, aktivierte oder nicht aktivierte Gerinnungsfaktoren, wie Faktor II, V, VII, VIII, IX, X, XIII, « FEIBA » und Prothrombinkomplexpräparationen, plasmatische Inhibitoren, Immunglobuline oder sonstige Blutprodukte, wie Fibronectin und Fibrinogen, enthalten.

Aus der veröffentlichten europäischen Patentanmeldung 0 018 561 ist es bekannt, Gerinnungsfaktoren enthaltende Präparationen, die praktisch fibrinogenfrei sind, gegen Hitzeeinwirkung zu stabilisieren, indem der Lösung der Gerinnungsfaktoren Aminosäure und ein Mono-, Oligosaccharid oder Zuckeralkohol zugesetzt wird. Nach einer solchen Behandlung kann die Präparation 10 h bei 60 °C erhitzt werden, wobei es bekannt ist, daß eine solche Behandlung bei Humanalbumin eine Inaktivierung von Hepatitisviren bewirkt. Das bekannte Verfahren hat den Nachteil, daß die Ausbeute an den Gerinnungsfaktoren nicht zufriedenstellend ist. Weiters wurde das Verfahren gegenüber Modellviren nicht erprobt, so daß das Wirkungsspektrum dieser Stabilisierungsbehandlung auf verschiedene Krankheitserreger unbekannt ist.

Nach der veröffentlichen europäischen Patentanmeldung 0 052 827 ist des weiteren ein Verfahren zur Herstellung einer Präparation der Gerinnungsfaktoren II und/oder VII bekannt geworden, welches hepatitissicher sein soll ; das Verfahren besteht im Erwärmen der Präparation in Gegenwart einer Aminosäure, eines Saccharids oder Zuckeralkohols und eines Chelatbildners, wie eines Salzes der Äthylen-diamino-tetraessigsäure.

Gemäß dem Verfahren nach der veröffentlichten europäischen Patentanmeldung 0 053 338 wird bei einem Verfahren zur Herstellung einer die Blutgerinnungsfaktoren IX und/oder X enthaltenden Präparation, die hepatitissicher sein soll, die Präparation in Gegenwart einer Aminosäure, eines Saccharids oder Zuckeralkohols und Ca-Ionen erwärmt, wobei den Ca-Ionen eine stabilisierende Wirkung gegen Hitzedenaturierung der Faktoren IX und X zugeschrieben wird.

In der veröffentlichten europäischen Patentanmeldung 0 035 204 ist weiters ein Verfahren zur Herstellung einer Proteinkomposition beschrieben, welches Verfahren darin besteht, daß die Komposition mit einem Polyol gemischt und die Mischung erhitzt wird, während einer Dauer, die ausreicht, um die Proteinkomposition zu pasteurisieren. Eine Modifikation dieses Verfahrens ist in der veröffentlichten europäischen Patentanmeldung 0 065 256 beschrieben, wobei eine Lösung von Plasminogen, die gegebenenfalls eine Aminosäure, ein Saccharid oder einen Zuckeralkohol enthält, in Gegenwart eines Proteinaseninhibitors erwärmt wird. Auch bei diesem Verfahren ist das Wirkungsspektrum der Behandlung nicht bekannt und die Ausbeute nicht zufriedenstellend.

Ein weiteres zum Stand der Technik gehörendes Verfahren ist in der US-PS 3 227 626 beschrieben, worin eine Plasminogenlösung in Gegenwart von Lysin 10 h bei 60 °C erhitzt wird, um die Präparation zu sterilisieren. Eine Wirksamkeit gegenüber einem bestimmten Virusspektrum ist auch dieser Literaturstelle nicht zu entnehmen.

In einer weiteren Literaturstelle, nämlich Fed. Proc. Vol. 41, 1982, Abstract 2877, Seite 763, ist beschrieben, daß ein $C_1$-Inaktivator (CI-INA) enthaltendes Konzentrat therapeutisch mit Vorteil verwendet werden kann, wenn es 10 h bei 60 °C in Gegenwart von Kalium- oder Ammoniumcitrat erhitzt wird, wobei diese Behandlung das Risiko einer Hepatitisübertragung herabsetzt.

Ein ähnliches Verfahren ist auch aus Journal of Biological Chemistry, Vol. 256, 1981, Nr. 23, Seite 12140, zur Stabilisierung einer Antithrombin III-Präparation bekannt geworden.

Aus der DE-OS 31 02 217 ist weiters bekannt, ein Plasminogenpräparat mit einem physiologisch verträglichen anorganischen Salz, Lysin, Phenylmethansulfonylfluorid, Aprotinin oder Sojabohnen-Trypsin-Inhibitor zu versetzen, doch werden bei diesem bekannten Verfahren die zugesetzten Salze bzw. Inhibitoren nicht entfernt, sondern bleiben im Endprodukt, was die Aktivität der Präparationen und ihre Verträglichkeit vermindert.

Bei allen diesen Verfahren ist der Nachteil vorhanden, daß das Wirkungsspektrum der Behandlung nicht bekannt und die Ausbeuten nicht zufriedenstellend sind.

Die Erfindung bezweckt die Vermeidung der geschilderten Schwierigkeiten. Sie beruht auf der neuen Erkenntnis, daß die Inaktivierung von Krankheitserregern in biologischen und pharmazeutischen Medien von in den Präparationen enthaltenen Proteinen gehemmt wird. Die Erfindung stellt sich die Aufgabe, diese Schutzwirkung der Proteine auf Krankheitserreger zu durchbrechen bzw. zu überwinden, — unter gleichzeitiger Erhaltung der biologischen Aktivität und der molekularen Integrität der Proteine —, um auf diese Weise sichere Blutprodukte-Präparationen zur Verfügung zu stellen. Die zum Stand der Technik gehörenden Maßnahmen haben diese Wirkung nicht. Im Gegenteil, einige davon haben eine zusätzliche stabilisierende Wirkung auf die Krankheitserreger.

Die Erfindung besteht bei einem Verfahren der eingangs bezeichneten Art darin, daß die Präparation durch Zusatz von Ammoniumsulfat auf eine Salzkonzentration von mehr als 0,5 molar gebracht und wärmebehandelt wird, worauf das Salz aus der Präparation entfernt wird.

Weitere Merkmale und bevorzugte Ausführungsformen des erfindungsgemäßen Verfahrens sind in den Unteransprüchen angeführt.

Die Erfindung umfaßt auch die durch das Verfahren erhaltene Präparation.

Wie schon erwähnt, ist eine der Grundlagen, auf der die Erfindung fußt, die Erkenntnis, daß die Schutzwirkung von Proteinen auf Krankheitserreger durch Ammoniumsulfat und Wärmebehandlung aufgehoben wird. Diese Tatsache ist in den folgenden Modellversuchen am Beispiel verschiedener Viren veranschaulicht.

Versuch 1

Poliomyelitis-Virus Typ I wurde einerseits in isotone Kochsalzlösung, andererseits in eine 5 %ige Plasmaproteinlösung eingebracht. Die beiden mit Poliovirus versetzten Lösungen wurden bei 45 °C 10 Stunden erhitzt und einer Virustiterbestimmung unterzogen. Die Werte in der folgenden Tabelle sind dekadische Logarithmen der $TCID_{50}$ pro 0,1 ml, wobei $TCID_{50}$ bedeutet, daß 50 % der Gewebekulturansätze einen cytopathischen Effekt zeigen.

| | Virustiter nach 10stündiger Erhitzung bei 45°C |
|---|---|
| Virus in isotoner Kochsalzlösung | 3,9 |
| Virus in Plasmaproteinlösung | 7,8 |

Der Versuch wurde mit dem gleichen Virus wiederholt, jedoch wurden beide Lösungen vor der Erhitzung mit Ammoniumsulfat (AMS) bis zur annähernden Salzsättigung versetzt (0,7 g Ammoniumsulfat pro ml), wobei ein pH-Wert von 7,3 resultierte. Nach 10stündiger Erhitzung bei 45 °C wurde der Virustiter wie folgt bestimmt.

| | Virustiter nach 10stündiger Erhitzung bei 45°C |
|---|---|
| Virus in AMS-hältiger isotoner Kochsalzlösung | 4,0 |
| Virus in AMS-hältiger Plasmaproteinlösung | < 3 |

Versuch 2

Poliomyelitis-Virus Typ I, Rotavirus und Coxsackie-Virus wurden jeweils einer Plasmaproteinlösung mit einem Gehalt von 54 mg Protein/ml zugesetzt. Zu je 10 ml dieser mit Viren versetzten Plasmaproteinlösungen wurden 7 g Ammoniumsulfat zugefügt und der pH-Wert auf 7,0 gestellt.

In Parallelversuchen wurden die virus- und ammoniumsulfathältigen Lösungen einmal während 10 Stunden auf 4 °C gehalten und einmal während 10 Stunden auf 60 °C erhitzt. Anschließend wurde das Salz aus den Proben durch Dialyse entfernt und eine Virustiterbestimmung vorgenommen. Die in der folgenden Tabelle angegebenen Werte sind dekadische Logarithmen der $TCID_{50}$/0,1 ml.

| | Virustiter | |
|---|---|---|
| | 10 h/4°C | 10 h/60°C |
| Poliomyelitis-Virus Typ I + AMS in Plasmaproteinlösung | 7,2 | < 1 |
| Rotavirus + AMS in Plasmaproteinlösung | 9,0 | < 1 |
| Coxsackie-Virus + AMS in Plasmaproteinlösung | 9,0 | < 1 |

Das erfindungsgemäße Verfahren wird nun im Zuge von Herstellungsverfahren verschiedener Präparationen, die Blutprodukte enthalten, durch Beispiele näher erläutert, wobei zur Veranschaulichung der Inaktivierungswirkung der erfindungsgemäß anzuwendenden Maßnahmen in einem bestimmten Stadium des Fraktionierungsprozesses absichtlich Viren zugefügt wurden.

## Beispiel 1

46 l frisch gefrorenes Plasma wurden bei 0 °C bis + 4 °C aufgetaut. Das entstandene Kryopräzipitat wurde durch Zentrifugieren abgetrennt und in 960 ml einer 0,1 %igen Tri-Natriumcitratlösung, bei 37 °C gelöst. Dieses Faktor VIII-hältige gelöste Kryopräzipitat wurde nach der in der US-PS 3 973 002 beschriebenen Methode auf einen pH-Wert von 6,0 bis 6,8 gestellt, wobei ein Niederschlag entstand, der durch Zentrifugieren abgetrennt und verworfen wurde. Diese Lösung wurde auf einen Faktor VIII-Gehalt von 20 Einheiten/ml eingestellt und mit Poliomyelitis-Virus Typ I versetzt ; sodann wurde Ammoniumsulfat (AMS) in einer Menge von 0,7 g/ml zugefügt und der pH-Wert auf 7,3 eingestellt. Dann wurde die Präparation in vier Teile geteilt und diese bei + 4 °C, 50 °C, 55 °C und 60 °C während 10 Stunden inkubiert. Anschließend wurde der Virustiter bestimmt.

Die Ergebnisse sind aus der folgenden Tabelle zu ersehen.

| | Behandlungs- temperatur | Virustiter |
|---|---|---|
| Faktor VIII-Präpara- tion + Virus + AMS | 4°C | 8 |
| Faktor VIII-Präpara- tion + Virus + AMS | 50°C | < 3 |
| Faktor VIII-Präpara- tion + Virus + AMS | 55°C | < 3 |
| Faktor VIII-Präpara- tion + Virus + AMS | 60°C | < 3 |

Man ersieht daraus, daß durch die erfindungsgemäße Salz- und Hitzebehandlung der Virustiter auf $< 10^3$ erniedrigt wurde.

## Beispiel 2

Einer wie in Beispiel 1 hergestellten Faktor VIII-Präparation, jedoch mit einem Proteingehalt von 7 mg/ml (2 E Faktor VIII/ml) wurde Poliomyelitis-Virus Typ I zugesetzt, dann Ammoniumsulfat in einer Menge von 0,8 g/ml zugefügt und diese Lösung während Zeiträumen von 2 Minuten, 6 Minuten, 20 Minuten, 3 Stunden und 10 Stunden bei 60 °C wärmebehandelt.

Ein Parallelversuch wurde durchgeführt, bei welchem jedoch vor dem Zusatz des Ammoniumsulfates Natriumcaprylat in einer Menge von 16 mmol/g Protein der Mischung zugefügt worden waren. Die Ergebnisse sind aus der folgenden Tabelle zu ersehen.

| Dauer der Wärmebehandlung bei 60°C | Virustiter einer Faktor VIII-Prä- paration + AMS | |
|---|---|---|
| | ohne Caprylat | mit Caprylat |
| Ausgangswert — | 6,8 | 6,0 |
| 2 Minuten | 6,3 | 6,8 |
| 6 Minuten | 6,2 | 5,8 |
| 20 Minuten | 5,7 | < 2,5 |
| 60 Minuten | 4,5 | < 2,5 |
| 3 Stunden | < 2 | < 2,5 |
| 10 Stunden | < 2 | < 2,5 |

Daraus ist ersichtlich, daß ohne Zusatz des Caprylates nach 3 Stunden eine deutliche Erniedrigung des Virustiters stattfindet ; mit Caprylatzusatz tritt diese Wirkung schon nach 20 Minuten ein.

In den folgenden Beispielen wird der weitere wichtige erfindungsgemäße Effekt, nämlich die Erhaltung der biologischen Aktivität der Faktor VIII-hältigen Präparationen nach durchgeführter Salz- und Wärmebehandlung bei variierender Konzentration des Salzes veranschaulicht.

### Beispiel 3

Je 10 ml der in beschriebener Weise hergestellten virushältigen Faktor VIII-Präparation mit 2 Einheiten Faktor VIII/ml wurden mit je 6,4 g, 8,0 g, 9,6 g Ammoniumsulfat versetzt. Der pH-Wert wurde auf pH 7,0 gestellt und die Mischungen solange gerührt, bis sich das Ammoniumsulfat vollständig gelöst hatte bzw. eine Sättigung von Ammoniumsulfat erreicht war. Die Lösungen wurden in ein Wasserbad gebracht und 10 Stunden bei 60 °C erhitzt. Kontrollansätze mit dem gleichen Gehalt an Faktor VIII-Einheiten und gleichem Ammoniumsulfatgehalt wurden keiner Hitzebehandlung unterworfen. Alle Proben wurden einer Dialyse gegen NaCl-NaCitratlösungen mit je 6 g/l unterzogen, um das Ammoniumsulfat mit Hilfe einer semipermeablen Membran zu entfernen. Anschließend wurden an allen Proben eine Faktor VIII-Aktivitätsbestimmung (2-Stufentestmethode in « A Laboratory Manual of Blood Coagulation », D.E.G. Austen, I.L. Rhymes ; Blackwell Scientific Publications 1975) und eine Virustiterbestimmung durchgeführt. Die Aktivitäten der hitzebehandelten Proben wurden mit den jeweils nicht hitzebehandelten ammoniumsulfatversetzten Kontrollansätzen verglichen und in % Restaktivität Faktor VIII ausgedrückt. Die Ergebnisse sind in der folgenden Tabelle angeführt.

| Faktor VIII-Präparation mit | % Restaktivität Faktor VIII | Virustiter |
|---|---|---|
| 6,4 g AMS/10 ml | 61 | < 2 |
| 8,0 g AMS/10 ml | 53 | < 2 |
| 9,6 g AMS/10 ml | 87 | < 2 |
| Kontrollproben | | 6,8 |

Es konnte in den mit AMS versetzten hitzebehandelten Proben keine Virusaktivität mehr nachgewiesen werden.

### Beispiel 4

Im folgenden Beispiel ist die Wirkung der erfindungsgemäßen Salz-Hitzebehandlung für verschiedene Proteinkonzentrationen gezeigt, wobei zu ersehen ist, daß die Wirkung über einen äußerst weiten Konzentrationsbereich erzielt wird.

Eine Faktor VIII-hältige Präparation wurde wie beschrieben hergestellt. Der Proteingehalt dieser Faktor VIII-Präparation wurde mit einer 0,1 %igen Trinatriumcitratlösung auf 1 mg Protein/ml bzw. 3 mg Protein/ml gestellt. Um eine Faktor VIII-hältige Präparation mit 100 mg Protein/ml herzustellen, wurde die beschriebene Faktor VIII-hältige Präparation mit einer hochkonzentrierten Plasmaproteinlösung (etwa 20 % Proteinlösung) versetzt, sodaß eine Faktor VIII-hältige Präparation mit 100 mg Protein/ml resultierte. Sodann wurde der Poliomyelitis-Virus, wie beschrieben, zugefügt.

Je 10 ml der Faktor VIII-hältigen Proteinlösung mit 1 mg bzw. 3 mg bzw. 100 mg Protein/ml wurden mit 8 g Ammoniumsulfat versetzt. Nach Lösen des Salzes wurden die Mischungen auf einen pH-Wert von 7,0 gestellt und 10 Stunden bei 60 °C erhitzt. Anschließend wurde das Ammoniumsulfat mittels Dialyse entfernt und die Restaktivität des Faktors VIII der Proben gegenüber den unbehandelten Proben bestimmt.

| Faktor VIII + AMS 10 Stunden 60°C in einer | % Restaktivität Faktor VIII | Virustiter |
|---|---|---|
| 0,1 %igen Proteinlösung | 13 | < 3 |
| 0,3 %igen Proteinlösung | 30 | < 3 |
| 10,0 %igen Proteinlösung | 55 | < 3 |

## Beispiel 5

In diesem Beispiel wird der Einfluß der Wärmebehandlung bei verschiedenen Temperaturen nach Zusatz von Ammoniumsulfat zu Faktor VIII-Präparationen gezeigt :

Je 10 ml der nach Beispiel 1 hergestellten virushältigen Faktor VIII-Präparation mit 2 E/ml wurden mit 7,2 g Ammoniumsulfat versetzt. Nachdem sich das Ammoniumsulfat gelöst hatte, wurde der pH-Wert auf 6,5 gestellt und die Mischungen während 10 Stunden bei variabler Temperatur erhitzt ; die Entfernung des Ammoniumsulfats bzw. die Faktor VIII-Aktivitätsbestimmungen erfolgten wie in Beispiel 4.

Die Aktivitäten der Ammoniumsulfat-hitzebehandelten Proben wurden mit der Aktivität der unbehandelten Probe verglichen und in % Restaktivität ausgedrückt. Die Ergebnisse sind in der folgenden Tabelle angeführt.

| Behandlung bei | % Restaktivität F.VIII | Virustiter |
|---|---|---|
| 45°C | 100 | < 2 |
| 50°C | 100 | < 2 |
| 55°C | 90 | < 2 |
| 60°C | 67 | < 2 |
| 62°C | 67 | < 2 |
| 66°C | 58 | < 2 |

Daraus ist ersichtlich, daß auch noch bei einer Temperatur von über 60 °C die Restaktivität in einem hohen Maße erhalten bleibt und das Virus inaktiviert wird.

## Beispiel 6

10 ml einer Faktor VIII-hältigen virushältigen Präparation mit 2 E Faktor VIII/ml wurden mit 8 g Ammoniumsulfat versetzt. Nach Auflösung des Ammoniumsulfats wurde der pH-Wert auf 7,0 gestellt und die Mischung 3 Minuten bei 90 °C erhitzt. Anschließend wurde Ammoniumsulfat mittels Dialyse entfernt und das hitzebehandelte Präparat einer Faktor VIII-Bestimmung unterzogen. Die Aktivität wurde mit der Aktivität der nicht behandelten Faktor VIII-hältigen Präparation verglichen und in % Restaktivität ausgedrückt.

So wurde festgestellt, daß auch bei einer relativ hohen Temperatur von 90 °C die Restaktivität noch zu 35 % erhalten blieb und der Virus seine Aktivität verliert. Der Virustiter ist $< 10^3$.

## Beispiel 7

In diesem Beispiel wird die pH-Abhängigkeit im Rahmen des erfindungsgemäßen Verfahrens dargestellt.

Je 10 ml einer Faktor VIII-hältigen virushältigen Präparation mit 7 mg/ml Protein und 2 E Faktor VIII/ml wurden mit je 7,2 g Ammoniumsulfat versetzt. Nach Auflösung des Salzes wurden die pH-Werte der einzelnen Mischungen wie folgt gestellt : pH 6,5, 7,0, 7,5, 8,0, 8,5, 9,0, 10,0. Die so pH-gestellten Proben wurden 10 Stunden bei 60 °C erhitzt. Anschließend erfolgte die Entfernung des Ammoniumsulfats mittels Dialyse und eine Faktor VIII-Bestimmung, die als % Restaktivität sich auf die unbehandelte Faktor VIII-hältige Probe ausdrückt und eine Virustiterbestimmung.

| | pH 6,5 | 7,0 | 7,5 | 8,0 | 8,5 | 9,0 | 10,0 |
|---|---|---|---|---|---|---|---|
| Virustiter | < 2 | < 2 | < 2 | < 2 | < 2 | < 2 | < 2 |
| % Restaktivität Faktor VIII nach 10 h 60°C mit Ammoniumsulfat | 65 | 60 | 65 | 55 | 60 | 35 | 25 |

Im nächsten Beispiel wird der Einfluß eines Glycinzusatzes zu einer Faktor VIII-hältigen Fraktion veranschaulicht.

## Beispiel 8

37 l frisch gefrorenes Plasma wurden bei 0 °C bis + 4 °C aufgetaut. Das entstandene Faktor VIII-hältige Kryopräzipitat wurde durch Zentrifugieren abgetrennt und mit 400 ml einer 0,1 %igen Trinatriumcitratlösung bei 37 °C gelöst. Die Lösung enthielt 66 mg Protein und 22 E Faktor VIII/ml. Der Lösung wurde ein Poliomyelitis-Virus Typ I zugesetzt.

Je 10 ml dieser Faktor VIII- und virushältigen Proteinlösung wurden

a) mit 8 g Ammoniumsulfat versetzt, der pH-Wert auf 7 eingestellt und die Lösung 10 Stunden bei 60 °C erhitzt ;

b) mit 0,05 g Glycin und 8 g Ammoniumsulfat versetzt, der pH-Wert auf 7 eingestellt und die Lösung 10 Stunden bei 60 °C erhitzt ;

c) mit 0,1 g Glycin und 8 g Ammoniumsulfat versetzt, der pH-Wert auf 7 eingestellt und die Lösung 10 Stunden bei 60 °C erhitzt ;

d) mit 0,5 g Glycin und 8 g Ammoniumsulfat versetzt, der pH-Wert auf 7 eingestellt und die Lösung 10 Stunden bei 60 °C erhitzt.

Anschließend wurde Ammoniumsulfat mittels Dialyse entfernt und die % Restaktivität Faktor VIII der hitzebehandelten Proben gegenüber den unbehandelten Proben bestimmt, ebenso der Virustiter bestimmt. Die Ergebnisse sind in der folgenden Tabelle angeführt.

| | Glycingehalt der Faktor VIII-Präparationen | | | |
| --- | --- | --- | --- | --- |
| | ohne Glycin | 0,5 % Glycin | 1 % Glycin | 5 % Glycin |
| Virustiter | < 2 | < 2 | < 2 | < 2 |
| % Restaktivität Faktor VIII nach AMS – 10 Stunden 60°C | 15 | 23 | 39 | 69 |

## Beispiel 9

2 ml einer in beschriebener Weise hergestellten Faktor VIII-Präparation mit einem Proteingehalt von 38 mg und 29,5 E Faktor VIII/ml wurden mit 1,6 g Ammoniumsulfat versetzt und der pH-Wert auf 7,0 gestellt und die Mischung aus Niederschlag und Lösung gefriergetrocknet. Die lyophilisierte Probe wurde 10 Stunden bei 60 °C erhitzt, mit Wasser rekonstituiert, einer Dialyse unterworfen und schließlich eine Faktor VIII-Aktivitätsbestimmung vorgenommen. Die Faktor VIII-Restaktivität betrug gegenüber einer nicht hitzebehandelten Kontrollprobe 34 %.

## Beispiel 10

Frisch gefrorenes menschliches Citratplasma wird aufgetaut und das dabei anfallende Kryopräzipitat abgetrennt. Dem Kryoüberstand wird DEAE-Sephadex® zugesetzt und während einer Kontaktzeit von 12 Stunden wird die FEIB-(Factor-Eight-Inhibitor-Bypass)-Aktivität gemäß der DE-OS 31 27 318 generiert. Alle Gerinnungsfaktoren inklusive FEIBA werden sodann vom DEAE-Sephadex mit einem Puffer eluiert. Nach einem Lyophilisationsschritt steht ein Bulkmaterial zur Verfügung. Von diesem Pulver werden 312 mg mit 10 ml $H_2O$ gelöst. Diese Lösung enthält 20 mg Protein, 24 E FEIBA und 28 E Faktor VII/ml.

Zur Veranschaulichung der Inaktivierungswirkung der erfindungsgemäß anzuwendenden Maßnahmen wurde nun diesem Produkt absichtlich ein Virus, nämlich ein Virus von Poliomyelitis Typ I zugefügt. Sodann wurde zu einer Probe des virusversetzten Produktes Ammoniumsulfat (AMS) in einer Menge von 0,8 g/ml zugefügt, während eine Kontrollprobe salzfrei blieb. Beide Proben wurden auf pH 7 gestellt. Die salzhältige Probe wurde 10 Stunden bei 60 °C erhitzt und anschließend bei beiden Proben der Virustiter bestimmt. Die Ergebnisse sind in der folgenden Tabelle angeführt.

(Siehe Tabelle Seite 8 f.)

|  | Virustiter | |
|---|---|---|
|  | Probe nach AMS Hitzeinaktivierung | Unbehandelte Kontrollprobe (+4°C) |
| Präparation enthaltend die Faktoren II, VII, IX, X, FEIBA | < 2,5 | 7,1 |

Die erfindungsgemäß kombinierte Salz-Hitze-Behandlung bewirkt nicht nur eine zuverlässige Inaktivierung von Krankheitserregern, wie Viren, sondern ein ebenso bedeutsamer Effekt ist die Erhaltung der biologischen Aktivität und der molekularen Integrität der Proteine. Um dies zu zeigen, wurde die wie im vorhergehenden beschriebene FEIBA-Präparation der Gerinnungsfaktoren einer Aktivitätsbestimmung vor und nach der Salz-Hitze-Behandlung unterzogen, wobei das AMS durch Dialyse entfernt wurde. Die Aktivitätsbestimmung erfolgte nach den in der genannten DE-OS 31 27 318 beschriebenen Methoden. Die Ergebnisse sind der folgenden Tabelle zu entnehmen.

|  | F.II | F.VII | F.IX | F.X | FEIBA |
|---|---|---|---|---|---|
| Restaktivität nach der AMS-Hitze-Behandlung, 0,8 g AMS/ml 10 h 60°C | 61 | 43 | 41 | 27 | 39 |

## Beispiel 11

Entsprechend der in Vox. Sang. 33, S. 37-50 (1977) beschriebenen Methode wurden die Gerinnungsfaktoren II, IX und X aus menschlichem Plasma über eine Adsorption an DEAE-Sephadex, eine Waschung des Anionenaustauschers und eine Elution des partiellen Prothrombinkomplexes gewonnen. Das Eluat wurde einem Dialyse- und Gefriertrocknungsprozeß unterworfen. Von diesem Bulkpulver wurden 467 mg mit 10 ml $H_2O$ gelöst. Diese Lösung enthielt 35 mg Protein, 70 E Faktor II, 52 E Faktor IX und 61 E Faktor X/ml und stellte somit eine Präparation eines partiellen Prothrombinkomplexes dar.

Wie vorher beschrieben, wurde der Präparation Poliomyelitis-Virus Typ I zugefügt und die Präparation mit AMS einer 10stündigen Hitzebehandlung bei 60 °C unterworfen. Anschließend erfolgte die Virustiterbestimmung und nach einer Dialyse die Bestimmung der Aktivität der Gerinnungsfaktoren. Die Ergebnisse sind in der folgenden Tabelle angeführt.

|  | Virustiter | |
|---|---|---|
|  | Probe nach AMS Hitzeinaktivierung | unbehandelte Kontrollprobe (+4°C) |
| Präparation enthaltend partiellen Prothrombinkomplex | < 2,5 | 6,6 |

|  | % Restaktivität nach AMS-Hitze-Behandlung | | |
|---|---|---|---|
|  | Aktivität Faktor II | IX | X |
| 0,8 g AMS/ml, 10 h 60°C | 66 | 39 | 28 |

0 124 506

## Beispiel 12

Entsprechend der in der AT-PS 359 646 beschriebenen Methode wurde eine Gerinnungsfaktor VII enthaltende Präparation aus menschlichem Citratplasma hergestellt. Nach Abtrennung des Kryopräzipitates und einer DEAE-Sephadex-Behandlung wurde Faktor VII an Al(OH)$_3$ adsorbiert. Al(OH)$_3$ wurde einem Waschprozeß und einem Faktor VII-Elutionsprozeß bei erhöhter Ionenstärke unterworfen. Das Faktor VIIhältige Eluat wurde dialysiert und lyophilisiert. 192 mg des Bulkpulvers wurden mit 10 ml H$_2$O gelöst. Diese Lösung enthielt 10,3 mg Protein und 21 E Faktor VII/ml. Wie vorher beschrieben, wurde auch hier absichtlich Poliomyelitis-Virus Typ I zugefügt und die Präparation mit AMS einer 10stündigen Hitzebehandlung bei 60 °C unterworfen. Anschließend erfolgte die Virustiterbestimmung und nach einer Dialyse die Bestimmung der Aktivität der Gerinnungsfaktoren. Die Ergebnisse sind in der folgenden Tabelle angeführt.

| | Virustiter | |
|---|---|---|
| | Probe nach AMS-Hitzeinaktivierung | unbehandelte Kontrollprobe (+4 °C) |
| Präparation enthaltend Faktor VII | $<3$ | 7,5 |

| | % Restaktivität nach AMS-Hitze-Behandlung |
|---|---|
| | Aktivität Faktor VII |
| 0,8 g AMS/ml 10 h 60°C | 58 |

## Beispiel 13

Eine Antithrombin III-Präparation wurde nach der in « Thrombosis Research 5, S. 439 (1974) » beschriebenen Methode affinitätschromatographisch über Heparin-Agarose aus humanem Plasma hergestellt und lyophilisiert. 530 mg dieses Antithrombin III-hältigen Bulkmaterials wurden in 10 ml Wasser gelöst. Diese Lösung enthielt 26 mg Protein und 25 E Antithrombin III pro ml.

Die Präparation wurde sodann mit Poliomyelitis-Virus Typ I versetzt. Dann wurde der Präparation Ammoniumsulfat in einer Menge von 0,8 g/ml zugefügt und der pH-Wert auf 7 gestellt. Dann wurde die Probe 10 Stunden bei 60 °C erhitzt, das Salz durch Dialyse entfernt und der Virustiter und die Aktivität des Antithrombin III in der Präparation amidolytisch gemessen. Die Bestimmungsmethode ist in Thrombosis Research 6, S. 287, 1975, beschrieben. Die Restaktivität wird in % gegenüber eine unbehandelten Probe ausgedrückt.

In der folgenden Tabelle sind die Virustiter in dekadischen Logarithmen der TCID$_{50}$/0,1 ml ausgedrückt ; die Restaktivität in Prozent.

| | Virustiter 10 h/60°C | | Restaktivität % |
|---|---|---|---|
| | ohne AMS | mit AMS | |
| Antithrombin III-Präparation | 6,9 | $<2,5$ | 71 |

## Beispiel 14

Eine C$_1$-Esterase-Inhibitor-Präparation wurde nach der in Vox. Sang. 26, S. 118 (1974) beschriebenen Methode aus humanem Plasma über Anionenaustauscher (DEAE-Sephadex) durch Adsorption und anschließende Elution gewonnen. Nach einer Salzfällung zur Abtrennung unerwünschter Proteine wurde

9

die gereinigte $C_1$-Inhibitor-Präparation gefriergetrocknet. 650 mg des $C_1$-Inhibitor-hältigen Bulkmaterials wurden mit 10 ml Wasser gelöst. 1 ml dieser Lösung enthielt 49 mg Protein und 60 E $C_1$INA.

Die Lösung wurde mit Poliomyelitis-Virus Typ I beimpft und sodann in erfindungsgemäßer Weise behandelt, indem 0,8 g Ammoniumsulfat/ml zugefügt, der pH-Wert auf 7 gestellt und die Probe 10 Stunden bei 60 °C erhitzt wurde. Nach Entfernung des Ammoniumsulfates mittels Dialyse wurde eine Virustiterbestimmung vorgenommen und die Restaktivität des $C_1$-Inhibitors mit chromogenem Substrat nach der in « Mikrozirkulation und Prostaglandinstoffwechsel », Verhandlungsbericht der 25. Tagung der Deutschen Arbeitsgemeinschaft für Blutgerinnungsforschung in München, Februar 1981, S. 221, F.K. Schattauer Verlag, Stuttgart — New York, beschriebenen Methode festgestellt.

In der folgenden Tabelle sind die Virustiterwerte, angegeben in dekadischen Logarithmen der $TCID_{50}/0,1$ ml, und die Restaktivität in % gegenüber einer unbehandelten Probe angegeben.

| | Virustiter 10 h/60°C | | Restaktivität % |
|---|---|---|---|
| | ohne AMS | mit AMS | |
| $C_1$-Esterase-Inhibitor-Präparation | 6,9 | < 2,5 | 85 |

### Beispiel 15

In diesem Beispiel wird der Einfluß der erfindungsgemäßen Behandlung an einer Plasmapräparation demonstriert. Plasma wurde mit Poliomyelitis-Virus Typ I versetzt, Ammoniumsulfat in einer Menge von 0,8 g/ml zugefügt, der pH-Wert auf 7 gestellt und die Probe einer Hitzebehandlung während 10 Stunden bei 60 °C, 65 °C und 75 °C unterworfen. Anschließend wurde das Ammoniumsulfat durch Dialyse entfernt, der Virustiter bestimmt und die Trypsin-Inhibition gemessen.

In der folgenden Tabelle sind die Virustiterwerte in dekadischen Logarithmen der $TCID_{50}/0,1$ ml angegeben und die Trypsin-Inhibition als % Restaktivität gegenüber einer unbehandelten Kontrolle zum Ausdruck gebracht.

| | Virustiter 10 h | | | | % Restaktivität an Trypsin-Inhibition | | |
|---|---|---|---|---|---|---|---|
| | Kontrolle | 60°C | 65°C | 75°C | 60°C | 65°C | 75°C |
| Plasma + AMS | | <2,5 | <2,5 | <2,5 | 62 | 62 | 53 |
| Plasma ohne AMS | 7 | | | | | | |

Die Methode der Bestimmung der Restaktivität besteht darin, daß 86,4 mg Trypsin (Pankreasprotease Merck Art. Nr. 8367) in 100 ml $10^{-3}$N HCl gelöst werden. Diese Trypsinlösung wird mit der zu bestimmenden Probe gemischt, wobei die im Plasma vorhandenen Trypsininhibitoren das zugesetzte Trypsin neutralisieren. Nicht neutralisiertes Trypsin wird amidolytisch mit chromogenem Substrat Bz-Ileu-Glu-Gly-Arg-pNA gemessen. Die verbliebene, nicht neutralisierte Trypsinmenge wird auf einen Trypsinleerwert, bei dem keine Probenzugabe erfolgte, bezogen. Sie stellt ein indirektes Maß für die Trypsininhibitoren dar.

### Beispiel 16

Eine Immunglobulin-Präparation wurde nach der Alkoholfraktionierungsmethode nach Cohn (Cohn Fraktion II) aus menschlichem Plasma gewonnen. Die Lösung enthielt 160 mg Protein mit einem Anteil von 97,3 % Gammaglobulin und 2,7 % $\alpha,\beta$-Globuline, 22,5 mg Glycin und 3 mg NaCl pro ml.

Die Lösung wurde sodann mit einem Poliomyelitis-Virus Typ I versetzt. Sodann wurden zu der Probe 0,8 g Ammoniumsulfat/ml zugesetzt, der pH-Wert auf 7 gestellt und die Präparation 10 Stunden bei 60 °C erhitzt. Anschließend wurde das Ammoniumsulfat mittels Dialyse entfernt und der Virustiter bestimmt.

Die Ergebnisse im Vergleich zu einer unbehandelten Probe sind aus der folgenden Tabelle ersichtlich, wobei die angegebenen Werte die dekadischen Logarithmen der $TCID_{50}/0,1$ ml sind.

|  | Virustiter | |
|---|---|---|
|  | 10 h/4°C | 10 h/60°C |
|  | ohne AMS | mit AMS |
| Immunglobulin-präparation | 5,7 | < 2,5 |

Um eventuelle Veränderungen an der erfindungsgemäß behandelten Immunglobulin-Präparation feststellen zu können, wurde an der mit Ammoniumsulfat und mit Hitze behandelten Probe eine elektrophoretische Auftrennung der Globuline vorgenommen, u. zw. auf einer Cellulose-Acetat-Membran bei einem pH-Wert von 8,6 und einer Ionenstärke von 0,075. Eine solche elektrophoretische Untersuchung ist in der AT-PS 368 883 beschrieben.

Die Ergebnisse der elektrophoretischen Auftrennung sind aus der folgenden Tabelle ersichtlich :

|  | % Protein | |
|---|---|---|
|  | $\alpha$,β-Globuline | $\gamma$-Globuline |
| Kontrolle ohne Hitze-behandlung | 2,7 | 97,3 |
| Probe mit AMS 10 h/60°C | 2,9 | 97,1 |

Es ist somit ersichtlich, daß keine Veränderung gegenüber der unbehandelten Probe stattgefunden hat, daß also die Proteine in ihrer molekularen Integrität und Mobilität unverändert geblieben sind.

Weiters wurde die erfindungsgemäß durch Ammoniumsulfat und Hitze behandelte Präparation noch einem Antikörpertitertest gegen Masern, Rubella und Pertussis unterzogen, um zu zeigen, daß während der AMS-Hitzebehandlung die Wirkung der Antikörper erhalten bleibt. Es wurden Hämagglutinationshemmtests und ein Agglutinationstitertest für die Bestimmung der Antikörper gegen Masern, Rubella bzw. Pertussis nach der in American Journal of Hygiene 71, S. 120 (1960) ; Journal Path. and Bact. 78 (1959) ; R. Trian in Recherches Immunologique 1965 Inst. Merieux beschriebenen Methode durchgeführt. Dabei wurde der Gehalt an Virus-neutralisierenden Antikörpern bestimmt. Viren rufen unter bestimmten Bedingungen eine Aggregation von Erythrocyten hervor. Diese agglutinierenden Eigenschaften der Virusantigene gehen durch die Reaktion mit spezifischen Antikörpern verloren. Um diesen Vorgang zu quantifizieren, wurde die Probe verdünnt und jede Verdünnung mit derselben Menge Virusantigen inkubiert. Der Endpunkt ist dann erreicht, wenn die Antikörper der Probe alle vorhandenen Antigene gebunden haben und keine Agglutination mit Erythrocyten eintritt. Die höchste Verdünnung, bei der diese Reaktion eintritt, ist der Endpunkt, wie in der folgenden Tabelle für Pertussis und Rubella angeführt. Bei Masernantikörper wird die Verdünnungsstufe der Probe noch mit der des Internat. WHO-Standards verglichen und als Internationale Einheit ausgedrückt.

|  | Antikörper | | |
|---|---|---|---|
|  | Masern IE/ml | Pertussis Verdünnungs-stufe | Rubella Verdünnungs-stufe |
| Kontrolle Immunglobulin | 12 | 1 : 142 | 1 : 168 |
| AMS 10 h 60°C | 12 | 1 : 142 | 1 : 200 |

Beispiel 17

Eine Plasminogen-Präparation wurde nach der von D. G. Deutsch und E. T. Mertz in Science 170, 1095 (1970) beschriebenen Methode hergestellt. 50 ml Lysin-Sepharose® wurden in einer Säule mit 0,1 M Phosphat, pH 7,4 äquilibriert. 340 ml Plasma wurden mit Wasser auf 640 ml verdünnt und die Säule wurde mit dieser Lösung beladen. Nach der Entfernung von Begleitproteinen durch Waschung mit einer 0,3 M Phosphatlösung (pH 7,4) wurde das Plasminogen mit 0,2 M 6-Aminocapronsäure (pH 7,4) eluiert. Die 6-Aminocapronsäure wurde durch Dialyse oder durch Gelfiltration über Sephadex G-25 entfernt und die Plasminogen-enthaltende Lösung wurde gefriergetrocknet.

230 mg dieses Bulkpulvers wurden in 10 ml Wasser gelöst. Diese Lösung enthielt 271 Casein-Einheiten Plasminogen und 13 mg Protein pro ml.

Die Präparation wurde, wie im vorhergehenden Beispiel beschrieben, mit Poliomyelitis-Virus Typ I versetzt, sodann wurden 0,8 g Ammoniumsulfat/ml zugefügt, der pH-Wert auf 7 gestellt und die Präparation sodann 10 Stunden bei 60 °C erhitzt. Anschließend wurde Ammoniumsulfat durch Dialyse entfernt und der Virustiter sowie die Aktivität von Plasminogen mit Urokinase und einem chromogenen Substrat (H-D-Val-Leu-Lys-pNA) nach der Methode in « Chromogenic Peptide Substrates : Chemistry and Clinical Usage », Edt. M. F. Scully, V. V. Kakkar, S. 128 (1979), Verlag Churchill Livingstone, bestimmt.

In der folgenden Tabelle ist der Virustiter und die Restaktivität gegenüber unbehandelten Kontrollproben dargestellt, wobei die angegebenen Werte des Virustiters die dekadischen Logarithmen der $TCID_{50}/0,1$ ml sind.

| | Virustiter | | Restaktivität |
| | 10 h/4°C | 10 h/60°C | % |
| | ohne AMS | mit AMS | |
| Plasminogen-präparation | 6,6 | < 2,5 | 55 |

## Beispiel 18

Eine $C_1$-Esterase-Präparation wurde nach der von D. H. Bing, J. M. Andrews, F. L. Suddath und R. Spencer, Prot. Biol. Fluids 23 551 (1975), Ed. H. Peeters, beschriebenen Methode hergestellt.

1 l Plasma wurde mit einer 50 %igen Lösung von Polyäthylenglykol (PEG) auf eine Endkonzentration von 5 % PEG gefällt. Der Niederschlag wurde nach der Abtrennung durch Zentrifugation mit Phosphat-gepufferter Kochsalzlösung (Ionenstärke 0,005) gewaschen und in 100 ml 0,5 M NaCl gelöst. Nach der Dialyse gegen isotone Kochsalzlösung, die 1 mmol/l $CaCl_2$ enthielt, wurde das clottierte Material entfernt. Eine 4 × 15 cm Säule wurde mit IgG-p-azobenzamidoäthylamino-Sepharose 6B gepackt und mit isotoner Kochsalzlösung, die 1 mmol/l $CaCl_2$ enthielt, äquilibriert. Diese Säule wurde mit der wie oben beschrieben hergestellten Plasmafraktion beladen und mit dem Äquilibrierungspuffer proteinfrei gewaschen. Unspezifisch gebundene Proteine wurden durch Waschung mit Borat-gepufferter Kochsalzlösung, die 1 mmol/l $CaCl_2$ enthielt, entfernt.

$C_1$-Esterase wurde mit einem Puffer eluiert, der 2,5 mmol/l EDTA enthielt. Das Eluat wurde gegen eine Tris-gepufferte Kochsalzlösung dialysiert und gefriergetrocknet.

4,5 g dieses Bulkpulvers wurden in 10 ml Wasser gelöst. 1 ml der Lösung enthielt 13,5 mg Protein.

Die Aktivität des Enzyms, bestimmt mit chromogenem Substrat (Pyroglutamyl-Gly-Arg-pNA), wurde amidolytisch gemessen und betrug 78,1 nmol pNA.$ml^{-1}$.$min^{-1}$ (Bestimmungsmethode gemäß « Mikrozirkulation und Prostaglandinstoffwechsel », 25. Tagung der Deutschen Arbeitsgemeinschaft für Blutgerinnungsforschung in München, Februar 1981, S. 221, F. K. Schattauer Verlag, Stuttgart-New York.

Die erhaltene Präparation mit den angegebenen Eigenschaften wurde sodann mit Poliomyelitis-Virus Typ I beimpft und sodann gemäß der Erfindung mit 0,8 g Ammoniumsulfat/ml versetzt, der pH-Wert auf 7 gestellt und während 10 Stunden auf 60 °C erhitzt. Nach der Behandlung wurde, wie beschrieben, der Virustiter und die amidolytische Aktivität festgestellt. In der folgenden Tabelle sind die Virustiterwerte und die Restaktivität in % gegenüber einer unbehandelten Kontrolle angegeben, wobei die angegebenen Werte des Virustiters die dekadischen Logarithmen der $TCID_{50}/0,1$ ml sind.

| | Virustiter | | Restaktivität |
| | 10 h/4°C | 10 h/60°C | % |
| | ohne AMS | mit AMS | |
| $C_1$-Esterase Präparation | 7,1 | < 2,5 | 63 |

**Patentansprüche**

1. Verfahren zur Inaktivierung von vermehrungsfähigen Krankheitsserregern in Präparationen, die plasmatische Enzyme und/oder Proenzyme, aktivierte oder nicht aktivierte Gerinnungsfaktoren, wie Faktor II, V, VII, VIII, IX, X, XIII, « FEIBA » und Prothrombinkomplexpräparationen, plasmatische Inhibitoren, Immunglobuline oder sonstige Blutprodukte, wie Fibronectin und Fibrinogen, enthalten, dadurch gekennzeichnet, daß die Präparation durch Zusatz von Ammoniumsulfat auf eine Salzkonzentration von mehr als 0,5 molar gebracht und wärmebehandelt wird, worauf das Salz aus der Präparation entfernt wird.

2. Verfahren nach Anspruch 1, dadurch gekennzeichnet, daß eine Präparation mit einem Proteingehalt von 0,001 bis 30 % eingesetzt wird.

3. Verfahren nach Anspruch 1 oder 2, dadurch gekennzeichnet, daß die Präparation mit einer 2- bis 4-molaren Ammoniumsulfatkonzentration wärmebehandelt wird.

4. Verfahren nach den Ansprüchen 1 bis 3, dadurch gekennzeichnet, daß aus der Präparation durch Zusatz von Ammoniumsulfat ein protein- und salzhältiger Niederschlag gewonnen und dieser der Wärmebehandlung unterworfen wird.

5. Verfahren nach den Ansprüchen 1 bis 3, dadurch gekennzeichnet, daß die ammoniumsulfathältige Präparation gefriergetrocknet und das Lyophilisat der Wärmebehandlung unterworfen wird, worauf durch Zusatz eines wässerigen Lösungsmittels zu dem Lyophilisat eine Lösung rekonstituiert und das Ammoniumsalz daraus entfernt wird.

6. Verfahren nach den Ansprüchen 1 bis 5, dadurch gekennzeichnet, daß die Wärmebehandlung während einer Dauer von 1 Sekunde bis 100 Stunden und bei einer Temperatur von 40 °C bis 121 °C durchgeführt wird.

7. Verfahren nach den Ansprüchen 1 bis 6, dadurch gekennzeichnet, daß die Wärmebehandlung als Schockbehandlung durchgeführt wird.

8. Verfahren nach den Ansprüchen 1 bis 7, dadurch gekennzeichnet, daß der pH-Wert der Präparation während der Behandlung in einem Bereich von 5 bis 11, vorzugsweise 6,5 bis 8,5, gehalten wird.

9. Verfahren nach den Ansprüchen 1 bis 8, dadurch gekennzeichnet, daß der Präparation vor der Wärmebehandlung proteinstabilisierende Substanzen, wie Glycin, zugesetzt werden.

10. Verfahren nach den Ansprüchen 1 bis 9, dadurch gekennzeichnet, daß der Präparation vor der Wärmebehandlung antimikrobielle Substanzen, wie Caprylate, zugesetzt werden.

11. Verfahren nach Anspruch 1, dadurch gekennzeichnet, daß die Entfernung des Ammoniumsulfates aus der Präparation mit Hilfe von semipermeablen Membranen erfolgt.

12. Präparation, enthaltend plasmatische Enzyme und Proenzyme, aktivierte oder nicht aktivierte Gerinnungsfaktoren, wie Faktor II, V, VII, VIII, IX, X, XIII, « FEIBA » und Prothrombinkomplexpräparationen, plasmatische Inhibitoren, Immunglobuline oder sonstige Blutprodukte, wie Fibronectin und Fibrinogen, erhältlich nach den Ansprüchen 1 bis 11, durch Behandeln dieser mittels Ammoniumsulfat bei einer Salzkonzentration von mehr als 0,5 molar sowie durch Wärmebehandlung und Entfernung des Salzes aus der Präparation und Haltbarmachen, insbesondere durch Lyophilisieren.

**Claims**

1. Method of inactivating reproducible pathogens in preparations containing plasmatic enzymes and/or proenzymes, activated or non-activated coagulation factors, such as Factors II, V, VII, VIII, IX, X, XIII, « FEIBA » and prothrombin complex preparations, plasmatic inhibitors, immunoglobulins or other blood products, such as fibronectin and fibrinogen, which method is characterised in that the preparation is brought to a salt concentration of more than 0.5 molar by addition of ammonium sulfate and is thermally treated, whereupon the salt is removed from the preparation.

2. Method according to claim 1, characterised in that a preparation with a protein content of from 0.001 % to 30 % is employed.

3. Method according to claim 1 or 2, characterised in that the preparation with a 2 to 4 molar ammonium sulfate concentration is thermally treated.

4. Method according to claims 1 to 3, characterised in that a protein and salt containing precipitate is recovered from the preparation by addition of ammonium sulfate, and that the same is thermally treated.

5. Method according to claims 1 to 3, characterised in that the ammonium sulfate containing preparation is freezedried and that the lyophilisate is thermally treated, whereupon, by addition of an aqueous solvent to the lyophilisate, a solution is reconstituted and the ammonium sulfate is removed therefrom.

6. Method according to claims 1 to 5, characterised in that the thermal treatment is carried out during a period of from 1 second to 100 hours and at a temperature of from 40 °C to 121 °C.

7. Method according to claims 1 to 6, characterised in that the thermal treatment is carried out as a shock treatment.

8. Method according to claims 1 to 7, characterised in that the pH of the preparation during treatment is maintained in a range of from 5 to 11, preferably in a range of from 6.5 to 8.5.

9. Method according to claims 1 to 8, characterised in that protein stabilising substances, such as glycine, are added to the preparation prior to the thermal treatment.

10. Method according to claims 1 to 9, characterised in that antimicrobial substances, such as caprylate, are added to the preparation prior to the thermal treatment.

11. Method according to claim 1, characterised in that the removal of ammonium sulfate from the preparation is effected with the help of semi-permeable membranes.

12. Preparation containing plasmatic enzymes and proenzymes, activated or non-activated coagulation factors, such as Factors II, V, VII, VIII, IX, X, XIII, « FEIBA » and prothrombin complex preparations, plasmatic inhibitors, immunoglobulins or other blood products, such as fibronectin and fibrinogen, obtainable according to claims 1 to 11 by treatment of the same with ammonium sulfate at a salt concentration of more than 0.5 molar as well as by thermal treatment and removal of the salt from the preparation and by rendering into stable form, in particular by lyophilisation.

**Revendications**

1. Procédé pour inactiver des germes pathogènes capables de proliférer dans des compositions contenant des enzymes et/ou proenzymes du plasma, des facteurs de coagulation activés ou non activés tels que les facteurs II, V, VII, VIII, IX, X, XIII, « FEIBA » et des compositions de complexes de prothrombine, des inhibiteurs du plasma, des immunoglobulines ou d'autres produits du sang tels que la fibronectine et le fibrinogène, caractérisé en ce que l'on règle la composition à une concentration en sel supérieure à 0,5 M par addition de sulfate d'ammonium et on la traite à la chaleur puis on élimine le sel de la composition.

2. Procédé selon la revendication 1, caractérisé en ce que l'on met en œuvre une composition à une teneur en protéines de 0,001 à 30 %.

3. Procédé selon la revendication 1 ou 2, caractérisé en ce que l'on traite à la chaleur la composition à une concentration en sulfate d'ammonium 2 M à 4 M.

4. Procédé selon les revendications 1 à 3, caractérisé en ce que l'on sépare de la composition, par addition de sulfate d'ammonium, un précipité contenant des protéines et des sels et on traite ce précipité à la chaleur.

5. Procédé selon les revendications 1 à 3, caractérisé en ce que la composition contenant le sulfate d'ammonium est lyophilisée et le lyophilisat est soumis au traitement à la chaleur, après quoi on reconstitue à partir du lyophilisat, par addition d'un solvant aqueux, une solution dont on élimine le sel d'ammonium.

6. Procédé selon les revendications 1 à 5, caractérisé en ce que le traitement à la chaleur est effectué en une durée de 1 seconde à 100 heures et à une température de 40 à 121 °C.

7. Procédé selon les revendications 1 à 6, caractérisé en ce que le traitement à la chaleur est réalisé sous forme d'un traitement de choc.

8. Procédé selon les revendications 1 à 7, caractérisé en ce que le pH de la composition, au cours du traitement, est maintenu dans un intervalle de 5 à 11, de préférence de 6,5 à 8,5.

9. Procédé selon les revendications 1 à 8, caractérisé en ce que l'on ajoute à la composition, avant le traitement à la chaleur, des substances qui stabilisent les protéines, par exemple de la glycine.

10. Procédé selon les revendications 1 à 9, caractérisé en ce que l'on ajoute à la composition, avant le traitement à la chaleur, des substances antimicrobiennes, par exemple des caprylates.

11. Procédé selon la revendication 1, caractérisé en ce que le sulfate d'ammonium est éliminé de la composition à l'aide de membranes semi-perméables.

12. Composition contenant des enzymes et pro-enzymes du plasma, des facteurs de coagulation activés ou non activés tels que les facteurs II, V, VII, VIII, IX, X, XIII, « FEIBA » et des compositions complexes de prothrombine, des inhibiteurs du plasma, des immunoglobulines ou d'autres produits du sang tels que la fibronectine et le fibrinogène, obtenue par un procédé selon les revendications 1 à 11, par traitement de la composition à l'aide du sulfate d'ammonium à une concentration en sel supérieure à 0,5 M, et par traitement à la chaleur et élimination du sel de la composition, et conservation, en particulier par lyophilisation.

14